# FASCICULE DE BREVET EUROPEEN

(11) **EP 1 178 794 B1**
(45) Date de publication et mention de la délivrance du brevet: **07.09.2005**
(21) Numéro de dépôt: 00927322.8
(22) Date de dépôt: 09.05.2000
(51) Int. Cl.: A61K 31/4035, A61P 29/00

(54) **UTILISATION DE DERIVES DE L'ACIDE SUCCINIQUE POUR OBTENIR UN MEDICAMENT DESTINE AU TRAITEMENT DE L'INFLAMMATION**
VERWENDUNG VON BERNSTEINSÄURE-DERIVATE FÜR DIE BEHANDLUNG VON ENTZÜNDUNGEN
USE OF SUCCINIC ACID DERIVATIVES TO OBTAIN A MEDICINE FOR TREATING INFLAMMATION

(30) Priorité: 11.05.1999 FR 9905978
(43) Date de publication de la demande: 13.02.2002
(73) Titulaire: Sanofi-Aventis, 75013 Paris (FR)
(72) Inventeur: CAILLE, Dominique, F-92190 Meudon (FR)
(74) Mandataire: Kugel, Dominique
(86) Numéro de dépôt international: PCT/FR2000/001246
(87) Numéro de publication internationale: WO 2000/067752

(56) Documents cités:
- EP-A- 0 507 534
- WO-A-96/34870
- WO-A-99/52876
- FR-A- 2 765 578

## Description

La présente invention a pour objet l'utilisation de dérivés de l'acide succinique, décrits dans le brevet EP 0 507 534, pour la préparation d'un médicament destiné au traitement de l'inflammation.

Ainsi la présente demande a pour objet l'utilisation des dérivés de l'acide succinique répondant à la formule générale (I) : 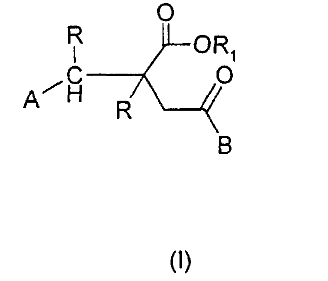 dans laquelle :
A représente un groupe phényle éventuellement substitué par un, deux ou trois substituants choisis parmi un halogène, un groupe C₁₋₆ alkyle, C₁₋₆ alcoxy; un thiényle, furyle, pyridyle ou un cycloalkyle ayant de 3 à 8 atomes de carbone;
B représente groupe aminobicyclique qui consiste en un composé cyclique amino à 5 ou 6 chaînons condensé avec un noyau cycloalkyle à 5 ou 6 chaînons qui peut avoir 1 ou 2 liaisons insaturées, avec la condition que B soit lié à l'atome de carbone du groupe carbonyle sur l'atome d'azote ; chaque R représente un atome d'hydrogène ou les restes R sont combinés ensemble pour former une liaison chimique ; R1 représente un atome d'hydrogène, un groupe C₁₋₆ alkyle, un groupe aralkyle ayant de 7 à 10 atomes de carbone ; quand il y a des isomères géométriques, chaque isomère géométrique, ses isomères E et ses isomères Z, ses isomères cis et ses isomères trans.

Les composés de formule générale (I) peuvent comprendre un ou plusieurs atomes de carbone asymétrique. Ils peuvent donc exister sous forme d'énantiomères ou de diastéréoisomères. L'utilisation de ces énantiomères, diastéréoisomères, ainsi que leurs mélanges, y compris les mélanges racémiques, fait partie de l'invention.

Les composés de formule générale (I) peuvent se présenter sous forme de base libre ou de sels d'addition à des acides pharmaceutiquement acceptables, tels que décrits dans EP0507534. L'utilisation de ces sels fait partie intégrante de la présente invention.

Dans la présente demande halogène représente un atome d'iode, chlore, brome ou fluor.

Plus particulièrement, l'utilisation des dérivés de l'acide succinique de formule (I) tels que définis ci-dessous: 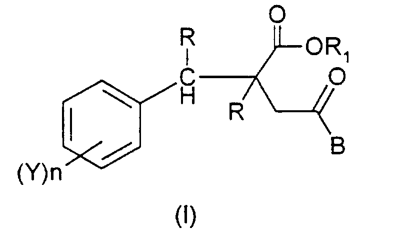 dans laquelle :
B représente groupe aminobicyclique qui consiste en un composé cyclique amino à 5 ou 6 chaînons condensé avec un noyau cycloalkyle à 5 ou 6 chaînons qui peut avoir 1 ou 2 liaisons insaturées, avec la condition que B soit lié à l'atome de carbone du groupe carbonyle sur l'atome d'azote ; chaque R représente un atome d'hydrogène ou les restes R sont combinés ensemble pour former une liaison chimique ; R1 représente un atome d'hydrogène, un groupe C₁₋₆ alkyle, un groupe aralkyle ayant de 7 à 10 atomes de carbone;
Y représente un atome d'hydrogène, un halogène, un groupe C₁₋₆ alkyle ou C₁₋₆ alcoxy et n représente 1, 2 ou 3 ; est préférée.

Parmi ces derniers, les composés préférés sont de formule (I): 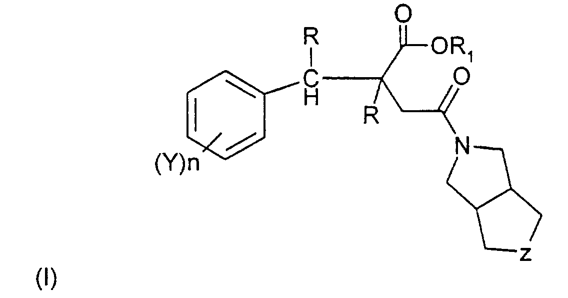 dans laquelle Z représente un groupe éthylène ou un groupe vinylène.

Plus spécifiquement, l'utilisation de l'acide 2-benzyl-3-(cis-hexahydro-2-isoindolinylcarbonyl)propionique de formule 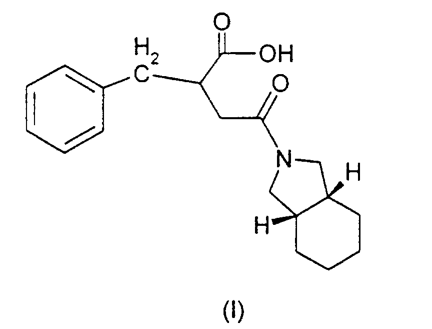 et plus particulièrement de l'acide (S)-2-benzyl-3-(cis-hexahydro-2-isoindolinylcarbonyl)propionique, est préférée.

Les composés de l'invention ont été soumis à des tests biologiques destinés à mettre en évidence leur activité anti-inflammatoire.

L'activité *in vivo* des composés de la présente invention a été étudiée dans un modèle expérimental d'inflammation plantaire chez le rat.

L'oedème inflammatoire de la patte de rat induit par l'injection intradermique de carraghénine (CAR) (1 %, v/v) est réalisé et évalué selon la méthode de WINTER C.A., et RISLEY E.A.(Carrageenan-induced edema in the hindpaw of rats as an assay for anti-inflammatory drugs. Proc. Soc. Axp. Biol. Med, 19632,11,544-547).

Les composés de l'invention sont donnés oralement 1 heure avant l'injection de CAR. Une solution de CAR à 1% dans une solution saline est injectée par voie s.c. dans la partie sous-plantaire de la patte postérieure droite de rat.

Le volume de la réaction inflammatoire est mesuré par plethysmographie après 1,5; 3 et 4,5 heures de l'injection de CAR.

Les composés de l'invention à des doses comprises entre 0,5 et 10 mg/kg par voie orale, confèrent une inhibition durable de l'inflammation induite (entre 1,5; 3 et 4,5 heures après l'injection de CAR) de l'ordre de 20 à 90 % par rapport au contrôle. De préférence, les composés de l'invention présentent à des doses de 10mg/kg une inhibition de l'inflammation de l'ordre de 50 à 90%.

Les résultats montrent que les composés de l'invention présentent *in vivo* des propriétés anti-inflammatoires. Ils peuvent donc être utilisés dans le traitement de symptomatique des affections douloureuses d'intensité légère à modérée et/ou des états fébriles, plus particulièrement dans les neuropathies du diabétique, les polyarthrites, les arthroses, les lombalgies, les douleurs traumatologiques et les inflammations dans le domaine ORL.

Les composés de l'invention peuvent être présentés, en association avec tout excipient approprié, sous toute forme convenant à une administration par voie orale ou parentérale, par exemple, sous forme de comprimés, des gélules, des dragées, ou des solutions buvables ou injectables, telle que définie dans EP0507534.

Les composés de l'invention peuvent être administrés à des doses quotidiennes comprises entre environ 1 et 100 mg chez l'adulte par voie orale, ou entre environ 0,1 et 100 mg par voie parentérale.

## Revendications

1. Utilisation d'un composé de formule (I) 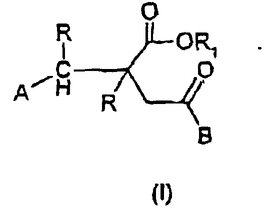 dans laquelle:
A représente un groupe phényle éventuellement substitué par un, deux ou trois substituants choisis parmi un halogène, un groupe C₁₋₆ alkyle, C₁₋₆ alcoxy; un thiényle, furyle, pyridyle ou un cycloalkyle ayant de 3 à 8 atomes de carbone;
B représente un groupe aminobicyclique qui consiste en un composé cyclique amino à 5 ou 6 chaînons condensé avec un noyau cycloalkyle à 5 ou 6 chaînons qui peut avoir 1 ou 2 liaisons insaturées, avec la condition que B soit lié à l'atome de carbone du groupe carbonyle sur l'atome d'azote ; chaque R représente un atome d'hydrogène ou les restes R sont combinés ensemble pour former une liaison chimique ; R1 représente un atome d'hydrogène, un groupe C₁₋₆ alkyle, un groupe aralkyle ayant de 7 à 10 atomes de carbone ; éventuellement son isomére E, ou son isomère Z, son isomère cis ou son isomère trans; éventuellement sous la forme d'un énantiomère, diastéréoisomère ou d'un mélange de ces différentes formes, y compris d'un mélange racémique, ainsi que les sels d'addition à des acides pharmaceutiquement acceptables de l'une de ces formes,
pour la fabrication d'un médicament destiné au traitement de l'inflammation.

2. Utilisation selon la revendication 1, **caractérisée en ce que** le composé de formule (I) est le composé : 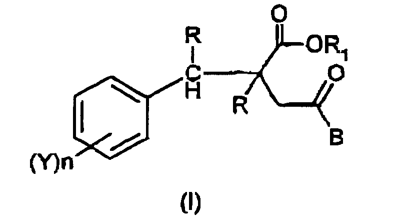 dans laquelle :
B représente groupe aminobicyclique qui consiste en un composé cyclique amino à 5 ou 6 chaînons condensé avec un noyau cycloalkyle à 5 ou 6 chaînons qui peut avoir 1 ou 2 liaisons insaturées, avec la condition que B soit lié à l'atome de carbone du groupe carbonyle sur l'atome d'azote ; chaque R représente un atome d'hydrogène ou les restes R sont combinés ensemble pour former une liaison chimique; R1 représente un atome d'hydrogène, un groupe C₁₋₆ alkyle, un groupe aralkyle ayant de 7 à 10 atomes de carbone;
Y représente un atome d'hydrogène, un halogène, un groupe C₁₋₆ alkyle ou C₁₋₆ alcoxy et n représente 1, 2 ou 3.

3. Utilisation selon la revendication 1, **caractérisé en ce que** le composé de formule (I) est le composé 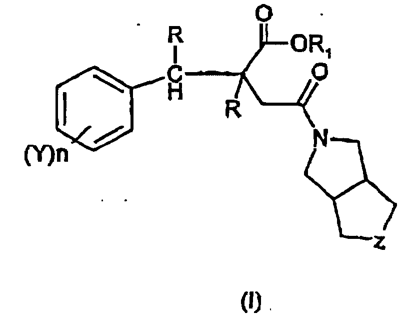 dans laquelle Z représente un groupe éthylène ou un groupe vinylène.

4. Utilisation selon la revendication 1, **caractérisé en ce que** le composé est l'acide (S)-2-benzyl-3-(cis-hexahydro-2-isoindolinylcarbonyl)propionique.

5. Utilisation selon l'une quelconque des revendications 1 à 4 **caractérisé en ce que** le médicament est destiné au traitement symptomatique des affections douloureuses d'intensité légère à modérée et/ou des états fébriles.

6. Utilisation selon l'une quelconque des revendications 1 à 4 **caractérisé en ce que** le médicament est destiné au traitement de neuropathies du diabétique, des polyarthrites, des arthroses, des lombalgies, des douleurs traumatologiques, des inflammations dans le domaine ORL.

## Patentansprüche

1. Verwendung einer Verbindung der Formel (I): 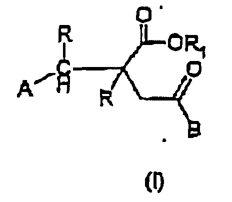 in der:
A eine Phenylgruppe, die gegebenenfalls durch einen, zwei oder drei Substituenten, ausgewählt aus einem Halogen, einer C₁₋₆-Alkylgruppe und einer C₁₋₆-Alkoxygruppe substituiert ist; eine Thienyl-, Furyl-, Pyridyl- oder Cycloalkyl-Gruppe mit 3 bis 8 Kohlenstoffatomen, bedeutet;
B eine aminobicyclische Gruppe bedeutet, welche aus einer cyclischen Aminoverbindung mit 5 oder 6 Kettengliedern, die mit einem Cycloalkylkern mit 5 oder 6 Kettengliedern, der 1 oder 2 ungesättigte Bindungen aufweisen kann, kondensiert ist, besteht, mit der Maßgabe, daß B über das Stickstoffatom an das Kohlenstoffatom der Carbonylgruppe gebunden ist:
jede der Gruppen R ein Wasserstoffatom bedeutet, oder die Reste R gemeinsam kombiniert sind zur Bildung einer chemischen Bindung;
R₁ ein Wasserstoffatom, eine C₁₋₆-Alkylgruppe oder eine Aralkylgruppe, die 7 bis 10 Kohlenstoffatome aufweist, bedeutet;
gegebenenfalls, ihres Isomeres E oder ihres Isomeres Z, ihres cis-Isomeres oder ihres trans-Isomeres;
gegebenenfalls in Form eines Enantiomeren, Diastereoisomeren oder einer Mischung dieser verschiedenen Formen, einschließlich einer racemischen Mischung, sowie der Additionssalze einer dieser Formen mit pharmazeutisch annehmbaren Säuren,
für die Herstellung eines Arzneimittels zur Behandlung von Entzündungen.

2. Verwendung nach Anspruch 1, **dadurch gekennzeichnet, daß** die Verbindung der Formel (I) folgende Verbindung ist: 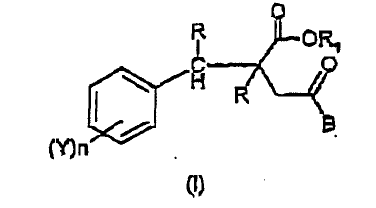 in der:
B eine aminobicyclische Gruppe bedeutet, welche aus einer cyclischen Aminoverbindung mit 5 oder 6 Kettengliedern, die mit einem Cycloalkylkern mit 5 oder 6 Kettengliedern, der 1 oder 2 ungesättigte Bindungen aufweisen kann, kondensiert ist, besteht, mit der Maßgabe, daß B über das Stickstoffatom an das Kohlenstoffatom der Carbonylgruppe gebunden ist:
jede Gruppe R ein Wasserstoffatom bedeutet oder die Reste R gemeinsam kombiniert sind zur Bildung einer chemischen Bindung;
R₁ ein Wasserstoffatom, eine C₁₋₆-Alkylgruppe oder eine Aralkylgruppe mit 7 bis 10 Kohlenstoffatomen bedeutet;
Y ein Wasserstoffatom, ein Halogenatom, eine C₁₋₆-Alkylgruppe oder eine C₁₋₆-Alkoxygruppe bedeutet und n 1, 2 oder 3 darstellt.

3. Verwendung nach Anspruch 1, **dadurch gekennzeichnet, daß** die Verbindung der Formel (I) die folgende Verbindung ist: 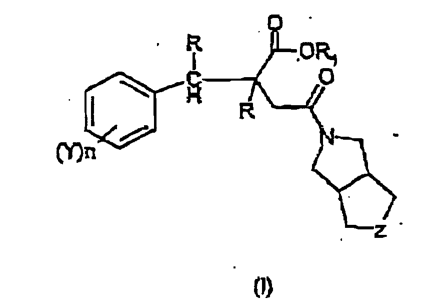 in der Z eine Ethylengruppe oder eine Vinylengruppe darstellt.

4. Verwendung nach Anspruch 1, **dadurch gekennzeichnet, daß** die Verbindung (S)-2-Benzyl-3-(cis-hexahydro-2-isoindolinylcarbonyl)-propionsäure ist.

5. Verwendung nach einem der Ansprüche 1 bis 4, **dadurch gekennzeichnet, daß** das Arzneimittel zur symptomatischen Behandlung von Schmerzen mit leichter oder mäßiger Intensität und/oder von Fieberzuständen bestimmt ist.

6. Verwendung nach einem der Ansprüche 1 bis 4, **dadurch gekennzeichnet, daß** das Arzneimittel bestimmt ist zur Behandlung von Nervenerkrankungen des Diabetikers, von Polyarthritis, Arthrosen, Lumbalgien, traumatologischen Schmerzen und Entzündungen im HNO-Bereich.

## Claims

1. Use of a compound of formula (I) 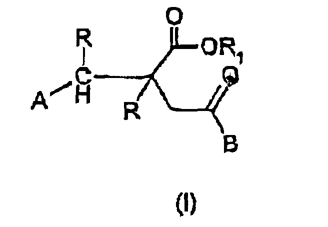 in which:
A represents a phenyl group optionally substituted by one, two or three substituents chosen from a halogen or a C₁₋₆ alkyl or C₁₋₆ alkoxy group; a thienyl, furyl or pyridyl or a cycloalkyl having from 3 to 8 carbon atoms;
B represents an aminobicyclic group which consists of a 5- or 6-membered cyclic amino compound condensed with a 5- or 6-membered cycloalkyl ring which can have one or two unsaturated bonds, with the condition that B is bonded to the carbon atom of the carbonyl group on the nitrogen atom;
each R represents a hydrogen atom or the R residues are combined together to form a chemical bond;
R₁ represents a hydrogen atom, a C₁₋₆ alkyl group or an aralkyl group having from 7 to 10 carbon atoms;
optionally its E isomers or its Z isomer, its cis isomers or its trans isomer; optionally in the form of an enantiomer or diastereoisomer or of a mixture of these various forms, including of a racemic mixture, and the addition salts with pharmaceutically acceptable acids of one of these forms,
in the manufacture of a medicament intended for the treatment of inflammation.

2. Use according to Claim 1, **characterized in that** the compound of formula (I) is the compound: 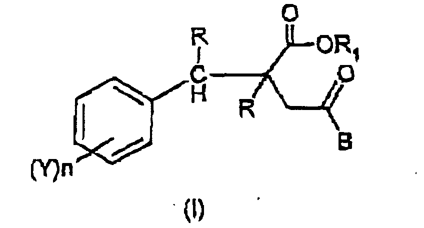 in which:
B represents an aminobicyclic group which consists of a 5- or 6-membered cyclic amino compound condensed with a 5- or 6-membered cycloalkyl ring which can have one or two unsaturated bonds, with the condition that B is bonded to the carbon atom of the carbonyl group on the nitrogen atom; each R represents a hydrogen atom or the R residues are combined together to form a chemical bond; R₁ represents a hydrogen atom, a C₁₋₆ alkyl group or an aralkyl group having from 7 to 10 carbon atoms;
Y represents a hydrogen atom, a halogen or a C₁₋₆ alkyl or C₁₋₆ alkoxy group and n represents 1, 2 or 3.

3. Use according to Claim 1, **characterized in that** the compound of formula (I) is the compound 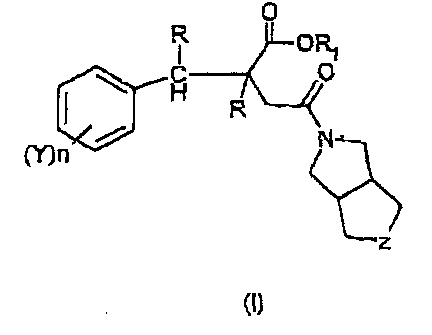 in which Z represents an ethylene group or a vinylene group.

4. Use according to Claim 1, **characterized in that** the compound is (S)-2-benzyl-3-(cis-hexahydro-2-isoindolinylcarbonyl)propionic acid.

5. Use according to any one of Claims 1 to 4, **characterized in that** the medicament is intended for the symptomatic treatment of painful conditions of light to moderate intensity and/or feverish states.

6. Use according to any one of Claims 1 to 4, **characterized in that** the medicament is intended for the treatment of diabetic neuropathies, polyarthritis, arthrosis, lumbago, traumatological pain and inflammation in the ENT field.
